Europäisches Patentamt

European Patent Office

Office européen des brevets

(11) Publication number: **0 277 008 B1**

## EUROPEAN PATENT SPECIFICATION

(45) Date of publication of patent specification: **14.06.95**

(51) Int. Cl.⁶: **C07D 498/04**, A61K 31/42, //(C07D498/04,263:00,205:00)

(21) Application number: **88300704.9**

(22) Date of filing: **27.01.88**

(54) **Process for crystallization of potassium clavulanate.**

(30) Priority: **29.01.87 US 8781**
    **17.07.87 US 74944**

(43) Date of publication of application:
    **03.08.88 Bulletin 88/31**

(45) Publication of the grant of the patent:
    **14.06.95 Bulletin 95/24**

(84) Designated Contracting States:
    **DE ES NL SE**

(56) References cited:
    **EP-A- 0 080 862**
    **GB-A- 1 504 425**
    **GB-A- 1 508 977**
    **GB-A- 2 003 863**
    **US-A- 4 454 069**

(73) Proprietor: **BEECHAM GROUP plc**
    **Four New Horizons Court**
    **Harlequin Avenue**
    **Brentford**
    **Middlesex TW8 9EP (GB)**

(72) Inventor: **Clark, Dennis Edward Beecham Laboratories**
    **101 Possumtown Road**

**Piscataway**
**New Jersey 08854 (US)**
Inventor: **Malik, Shaukat Hussain Beecham Laboratories**
**101 Possumtown Road**
**Piscataway**
**New Jersey 08854 (US)**
Inventor: **Butterly, Paul Gerard Beecham Laboratories**
**101 Possumtown Road**
**Piscataway**
**New Jersey 08854 (US)**
Inventor: **Badman, Clive Elton Beecham Pharmaceuticals**
**Clarendon Road**
**Worthing**
**West Sussex BN14 8OH (GB)**
Inventor: **Haseler, Jeffrey David Beecham Pharmaceuticals**
**Clarendon Road**
**Worthing**
**West Sussex BN14 8OH (GB)**

(74) Representative: **Lockwood, Barbara Ann et al**
**SmithKline Beecham plc**
**Corporate Intellectual Property**
**SB House**
**Great West Road**
**Brentford, Middlesex TW8 9BD (GB)**

## Description

The present invention relates to a novel process for the crystallisation of potassium clavulanate in crystalline rosettes having improved properties.

Clavulanic acid and its salts are described in British Patent Specification No. 1 508 977 (the contents of which is incorporated herein by reference thereto) as $\beta$-lactamase inhibitors capable of enhancing the antibacterial effects of $\beta$-lactam antibiotics, such as penicillins and cephalosporins. Antibacterial compositions comprising potassium clavulanate and amoxycillin are commercially available under the trade name 'Augmentin' (registered Trade Mark of Beecham Group p.l.c.), and certain oral dry unit-dose antibacterial compositions of potassium clavulanate and amoxycillin are described in British Patent Specification No. 2 005 538. Antibacterial compositions comprising potassium clavulanate and ticarcillin are commercially available under the trade name 'Timentin' (registered Trade Mark of Beecham Group p.l.c). Potassium clavulanate may also be formulated with other penicillins and cephalosporins to enhance their antibacterial efficacy, and may be formulated alone for separate co-administration with penicillins and cephalosporins.

Crystalline potassium clavulanate generally exists in the form of rod-like or needle-like crystals, which are generally relatively large, long crystals, sometimes agglomerated into plate-like crystals, and sometimes randomly aggregated into loosely formed bundles. This form of potassium clavulanate can give rise to processing difficulties in that the material does not always flow readily, is of low bulk density, and can be difficult to sieve. We have now found a process for the crystallisation of potassium clavulanate in another crystalline habit, i.e in the form of crystalline rosettes, having improved processing characteristics.

Crystalline potassium clavulanate in the form of crystalline rosettes each comprising a plurality of needle crystals radiating out from a common nucleation point is a known material, but only admixed solely with sodium ticarcillin in a ratio of 1 : 30 by weight, calculated as the free acid clavulanic acid : ticarcillin free acid, in the form of a sterile reconstitutable powder in a vial.

The present invention provides a process for the preparation of rosette-form crystalline potassium clavulanate, which comprises adding a solution of clavulanate ions to a non-solvent for potassium clavulanate, in the presence of potassium ions.

The rosette-form of crystalline potassium clavulanate, as defined above, has improved flow characteristics as compared with the standard needle-form potassium clavulanate. It also has improved sieving characteristics in that over 90% by weight of the material, generally over 95% by weight of the material, can readily be passed through an 850$\mu$m (number 20) mesh screen, whereas with the conventional needle form of the material generally about 30% by weight fails to pass through an 850$\mu$m mesh screen. Consequently, the rosette form of the material has considerable advantages for pharmaceutical processing and formulation.

According to one aspect of the process of the present invention, the rosette-form of crystalline potassium clavulanate is provided in substantially pure form, that is to say that the said rosette material contains not more than 50% by weight, advantageously not more than 25% by weight, preferably not more than 20% by weight, more preferably not more than 15% by weight, especially not more than 10% by weight, more especially not more than 5% by weight, yet more especially not more than 2% by weight, and very especially not more than 1% by weight of other crystalline forms or habits of potassium clavulanate, especially the conventional needle form (all percentages being based on the total weight of the material).

The rosette form of potassium clavulanate produced by the process according to the present invention may have a bulk density within the range of from 0.2 to 0.8 g/cm$^3$, advantageously from 0.2 to 0.6 g/cm$^3$, such as from 0.2 to 0.5 g/cm$^3$, for example from 0.3 to 0.5 g/cm$^3$.

In the rosette-form material produced by the process according to the present invention, generally at least 80%, advantageously at least 90%, preferably at least 95%, and more preferably at least 99%, by volume of the material has a particle size exceeding 1280 $\mu$m$^2$, with generally at least 65%, advantageously at least 75%, preferably at least 80%, and more preferably at least 90%, by volume of the material having a particle size exceeding 5120 $\mu$m$^2$. In contrast thereto, with needle-form material, generally at least 50%, and normally at least 60%, by volume of the material has a particle size below 1280 $\mu$m$^2$, with generally at least 75%, and normally at least 85%, by volume of the material having a particle size below 5120 $\mu$m$^2$. The particle size of the material may conveniently be determined by image analysis.

In the rosette-form material produced by the process according to the invention the individual rosettes are thought to consist of a plurality of small needle crystals radiating out from a common nucleation point: radiating clusters of crystals. This appears to be borne out by the fact that X-ray diffractograms show no significant difference in crystal structure between the rosette material and the standard needle-form material.

The individual needles in the rosettes may be extremely small and fine and may not be microscopically distinguishable under even 100-fold magnification: the rosettes may appear simply as small 'roses' or

3

spheres with little or no visible surface crystal growth. In other cases a small amount of needle-like crystal growth will be microscopically visible at the circumference of the rosettes, and in some cases the rosette will appear as a hard core with a large number of visible small radiating needles. In all cases, however, the rosette form of the material is clearly discernible microscopically and is clearly distinguishable from the standard needle-form material, which, under 100-fold magnification, clearly appears as such, for example as randomly arranged long thin needles.

Thus the particular crystalline habit of any given sample of potassium clavulanate can readily be ascertained simply by microscopic examination under, say, 100-fold magnification.

In the rosettes, the individual needle crystals may radiate out from the common nucleation point in a single plane or in a plurality of planes. Also, they may radiate out in certain directions only or in all directions. Thus, the individual needles may radiate out from the central nucleation point to form, for example, a full or part circle or a full or part sphere. Two or more individual rosettes may, of course, agglomerate together.

In many cases, particularly when the rosette surface has microscopically visible surface growth, the rosettes can be broken down (for example by trituration with liquid paraffin in an agate pestle and mortar) at least partially into the constituent individual fine needle crystals. In such cases, it is apparent both microscopically and by particle size determination that the constituent needles are much smaller and more fine than the needles of standard needle-form material. For example, in cases where it is possible to break the rosettes down substantially, generally at least 75% by volume of the broken-down material will have a particle size below $1280\mu m^2$, with generally at least 95% of the material having a particle size below $5120\mu m^2$.

In some cases, particularly when the rosettes appear microscopically simply as small 'roses' or spheres with no visible surface growth, the rosettes may be too hard and compact to be broken down in the manner described above. Nevertheless, X-ray diffractograms still show the basic crystal form to be essentially needle-like and it is still apparent from microscopic inspection that the crystal habit is fundamentally different from that of standard needle-form material.

It has been found that the precise form of the rosettes - that is to say, for example, whether or not there is visible surface crystal growth, whether the rosettes are hard or soft, whether they are compact or more open, whether they are largely full or partial rosettes, and whether they are agglomerated - depends, inter alia, on the precise method used for their preparation. In all cases, however, it is clearly apparent from microscopic inspection, and also from particle size determination, that the material is in rosette form.

The process of the present invention is advantageously effected at a temperature not exceeding 15°C.

The clavulanate ions may suitably be provided in the form of potassium clavulanate, in which case that will also be the source of the potassium ions. In this case, the process will be a crystallisation or recrystallisation process.

The form of the potassium clavulanate used as starting material for the crystallisation or recrystallisation is not critical and it may for example be needle-form crystalline material, rosette-form crystalline material, or amorphous material. It should advantageously be of high purity.

The clavulanate ions may alternatively be provided in the form of another clavulanate salt, for example tert-butylamine clavulanate. In this case, the potassium ions will be provided from another source and may be provided in the clavulanate solution or, advantageously, in the precipitating diluent (that is to say, the non-solvent for potassium clavulanate). The potassium ions may, for example, conveniently be provided in the form of potassium ethyl hexanoate.

The potassium ions should, of course, be present in an at least equivalent amount with respect to the clavulanate ions. This criteria will, of course, be satisfied when the potassium ions are provided by potassium clavulanate. When using another clavulanate salt with a separate source of potassium ions, the potassium ions are preferably present in excess, for example up to 1.5 equivalents, with respect to the clavulanate ions.

In order to reliably obtain the rosette form the precipitation should be effected by so-called 'inverse' or 'reverse' precipitation, that is to say the clavulanate solution is added to the precipitating diluent, in contrast to the 'normal' precipitation procedure in which the precipitating diluent is added to the solution of the material to be crystallised.

The solvent used for dissolution of the clavulanate ions may be any convenient solvent in which the particular clavulanate salt is soluble and which is compatible with the chosen precipitating diluent. Suitable solvents are especially aqueous alcohols, preferably an aqueous alkanol, more preferably an aqueous ($C_1$-$_6$)alkanol, for example aqueous methanol, aqueous ethanol, or aqueous isopropanol, as well as mixtures of two or more thereof, for example aqueous methanol/isopropanol. The aqueous content of the aqueous alcohol is suitably from 5 to 25% by volume.

The diluent used for precipitation of the potassium clavulanate may be any convenient non-solvent for potassium clavulanate which is compatible with the chosen dissolution solvent. Advantageously, the precipitating diluent is isopropanol or a mixture of isopropanol and acetone (up to 10 : 90 by volume).

Suitable combinations of solvent and precipitating diluent may readily be ascertained by simple trial experiments if necessary.

One method that has been found particularly suitable is to dissolve potassium clavulanate in aqueous methanol (suitably 75% to 95% methanol, preferably about 80% methanol) and then to initiate precipitation by addition of the resulting solution to a mixture of isopropanol and acetone (suitably about 3 volumes of isopropanol to 1 volume of acetone), according to the reverse precipitation procedure. According to one embodiment of this method, the precipitation may be carried out at a temperature within the range of from 0 to 10°C, and according to another embodiment of this method the precipitation may be carried out at a temperature within the range of from 10 to 15°C.

An alternative method involves recrystallisation of potassium clavulanate by dissolution in aqueous ethanol, followed by reverse precipitation in isopropanol/acetone (preferably about 3:1).

It is also possible to obtain the rosette-form material by dissolution of potassium clavulanate in aqueous isopropanol (preferably about 80-85% isopropanol) followed by reverse precipitation in isopropanol or with isopropanol/acetone (preferably about 3:1).

Another particularly suitable method for obtaining the desired rosette-form material is to dissolve tert-butylamine clavulanate in an aqueous solvent selected from isopropanol, methanol and mixtures thereof (suitably up to 25% by volume of water in each case), followed by addition of the resulting solution to a solution of potassium ions (suitably as potassium ethyl hexanoate) in a solvent selected from isopropanol and isopropanol/acetone (up to 90% by volume of acetone) so as to cause precipitation of potassium clavulanate in the form of crystalline rosettes.

In one embodiment of this method, tert-butylamine clavulanate is dissolved in aqueous methanol or in aqueous methanol/isopropanol (from 5 to 25% by volume of water in each case), followed by addition of the resulting clavulanate solution to a solution of potassium ethyl hexanoate in isopropanol or in isopropanol/acetone (up to 90% by volume of acetone).

In a second embodiment of this method, a solution of tert-butylamine clavulanate in aqueous isopropanol (from 2 to 10%, preferably about 5%, by volume of water) is prepared, and the resulting clavulanate solution is added to a solution of potassium ethyl hexanoate in isopropanol.

In all cases the pH of the clavulanate solution is advantageously 6.5 to 7.0 preferably slightly below 7.0, suitably from 6.5 to 6.9. Where necessary, the pH may suitably be adjusted by the addition of, for example, acetic acid.

It can also be advantageous to treat the clavulanate solution with decolourising charcoal, with subsequent filtration, prior to precipitation.

Precipitation is advantageously effected at a temperature not exceeding 15°C, advantageously from 0°C to 15°C, more advantageously from 5°C to 15°C, preferably from 8°C to 15°C, and especially from 10°C to 15°C. It has been found that the use of such a temperature is advantageous in consistently giving the desired rosette-form of the product.

The rosette-form material produced according to the process of the present invention may be dried, processed and formulated in a manner conventional for potassium clavulanate, but with the particular advantages discussed above.

Further details of formulating potassium clavulanate into pharmaceutical compositions, as well as details of dosages, and details of other antibacterially active $\beta$-lactam compounds for co-use with potassium clavulanate are given in British Patent Specification No. 1 508 977 and such details are also applicable to the rosette-form of the material according to the present invention.

The following examples and the accompanying figures illustrate the present invention.

Figs. 1 - 5, 8 and 10 show microphotographs of various samples of rosette-form potassium clavulanate, as further explained in the examples;

Figs. 6, 9 and 11 show microphotographs of the samples of Figs. 5, 8 and 10 subsequent to the rosettes being broken down, as further explained in Examples 2, 7 and 8; and

Fig. 7 shows, for comparison purposes, a microphotograph of a sample of the conventional crystalline form of potassium clavulanate in large individual needles, which are in some cases randomly formed into loosely bound aggregates, as further explained in the Comparison Example.

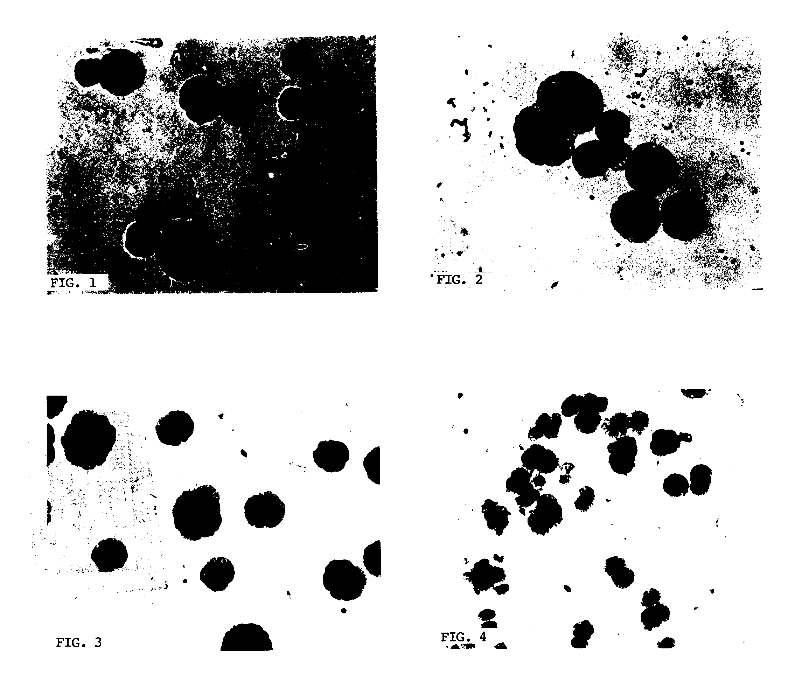

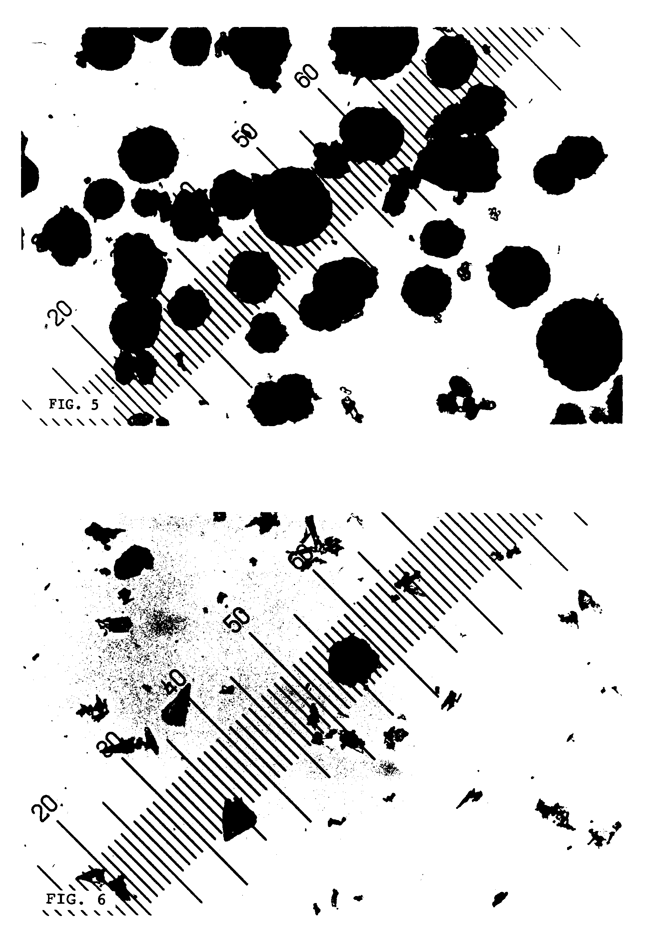

In Figs. 1 to 4, the magnification of the microphotographs is approximately 100-fold.

In Figs. 5 to 11, 1 scale division denotes 15μm.

In the examples and comparison examples, the particle sizes of the products were determined using an AMS (Analytical Measuring Systems) System III Image Analyser, using a Zeiss Lens with Plan 6.3.

5

**Example 1**

24 g of potassium clavulanate is added to a mixture of methanol (50 ml), glacial acetic acid (0.37 ml) and water (11 ml) at 18-20°C and stirred for 10 minutes at 18-20°C. The pH of the solution is checked to confirm it as 6.5 - 6.9. 2.4 g of charcoal (Norit) is added to the solution. The solution is stirred for 20 minutes, and then filtered over filter-aid into a 125 ml dropping funnel. The filtrate is added to 600 ml isopropanol/acetone (3:1) at 10-15°C over 20 minutes. The filter funnel and dropping funnel are washed with 10% $H_2O$/MeOH (25 ml) and the wash added to the suspension. The suspension is stirred for 1 hour at 10-15°C and then for 1 hour at 0-5°C. The product is filtered, washed with two portions of acetone (50 ml each), and dried in a vacuum oven [120-125 Pa (27-28 inches Hg)] with a dry air bleed at 25°C overnight.

The following data summarises results obtained by carrying out the above recrystallisation procedure on a number of samples of potassium clavulanate:

| | |
|---|---|
| Weight: | 21.6 g (average) |
| Assay: | 82-84% (from input assay of 80-84%; theoretical assay 83.9%) |
| Yield: | 89-92% |
| Moisture Content: | 0.5% |
| Crystal form: | Rosettes |
| Bulk density: | 0.29-0.46 g/cm$^3$ |

The accompanying Figs. 1 to 4 show microphotographs (100-fold magnification) of four samples of potassium clavulanate recrystallised by the above procedure.

**Example 2**

A further sample of potassium clavulanate was recrystallised using the procedure of Example 1. The product was of rosette crystal form and had a bulk density of 0.53 g/cm$^3$. Fig. 5 shows a microphotograph of the product.

The particle size of two samples of the resulting rosette material was determined by image analysis. The results are given in Table 1.

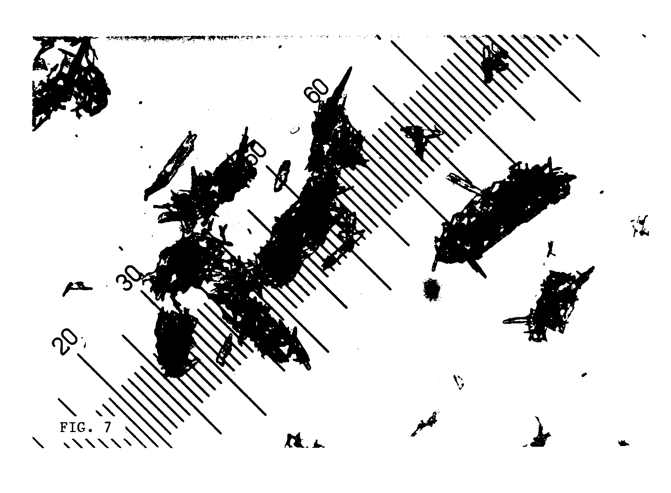

A portion of the rosette material was triturated with liquid paraffin in an agate pestle and mortar in order to break down the rosette crystal structure. Fig. 6 shows a microphotograph of the broken-down material, from which it can clearly be seen that the rosettes comprise clusters of radiating fine needles.

The particle size of the broken-down material was determined and the results are given in Table 2.

Table 1

| Particle size of rosette material (Example 2) | | | | |
|---|---|---|---|---|
| Particle size range ($\mu m^2$) | Volume % within range | | Volume % within range and above | |
| | Sample 1 | Sample 2 | Sample 1 | Sample 2 |
| 160 - 320 | 0.44 | 0.48 | 99.95 | 99.96 |
| 320 - 640 | 0.85 | 0.75 | 99.51 | 99.48 |
| 640 - 1280 | 1.06 | 1.07 | 98.66 | 98.73 |
| 1280 - 2560 | 1.37 | 2.64 | 97.60 | 97.66 |
| 2560 - 5120 | 8.38 | 12.00 | 96.23 | 95.02 |
| 5120 - 10240 | 36.45 | 36.01 | 87.85 | 83.02 |
| 10240 - 20480 | 45.23 | 41.13 | 51.40 | 47.01 |
| 20480 - 40960 | 6.17 | 5.88 | 6.17 | 5.88 |
| >40960 | 0.00 | 0.00 | 0.00 | 0.00 |

Table 2

| Particle size of broken rosette material (Example 2) | | |
|---|---|---|
| Particle size range ($\mu$m$^2$) | Volume % within range | Volume % within range and above |
| 10 - 20 | 5.25 | 99.96 |
| 20 - 40 | 10.73 | 94.71 |
| 40 - 80 | 17.21 | 83.98 |
| 80 - 160 | 19.62 | 66.77 |
| 160 - 320 | 19.08 | 47.15 |
| 320 - 640 | 9.44 | 28.07 |
| 640 - 1280 | 11.44 | 18.63 |
| 1280 - 2560 | 7.19 | 7.19 |
| >2560 | 0.00 | 0.00 |

**Example 3**

A further sample of potassium clavulanate was recrystallised using the procedure of Example 1. The product was of rosette crystal form and had a bulk density of 0.25 g/cm$^3$.

The particle size of two samples of the resulting rosette material was determined by image analysis. The results are given in Table 3.

A portion of the rosette material was triturated with liquid paraffin in an agate pestle and mortar in order to break down the rosette crystal structure. The particle size of the broken-down material was determined and the results are given in Table 4.

Table 3

| Particle size of rosette material (Example 3) | | | | |
|---|---|---|---|---|
| Particle size range ($\mu$m$^2$) | Volume % within range | | Volume % within range and above | |
| | Sample 1 | Sample 2 | Sample 1 | Sample 2 |
| 160 - 320 | 0.66 | 0.74 | 99.96 | 99.99 |
| 320 - 640 | 1.54 | 1.70 | 99.30 | 99.25 |
| 640 - 1280 | 2.88 | 3.50 | 97.76 | 97.55 |
| 1280 - 2560 | 5.63 | 6.25 | 94.88 | 94.02 |
| 2560 - 5120 | 9.18 | 9.63 | 89.25 | 87.77 |
| 5120 - 10240 | 19.54 | 18.09 | 80.07 | 78.14 |
| 10240 - 20480 | 27.07 | 25.40 | 60.53 | 60.05 |
| 20480 - 40960 | 29.87 | 28.74 | 33.46 | 34.65 |
| 40960 - 81920 | 3.59 | 5.91 | 3.59 | 5.91 |
| >81920 | 0.00 | 0.00 | 0.00 | 0.00 |

7

Table 4

| Particle size of broken rosette material (Example 3) | | |
|---|---|---|
| Particle size range ($\mu$m$^2$) | Volume % within range | Volume % within range and above |
| 10 - 20 | 0.80 | 99.96 |
| 20 - 40 | 1.64 | 99.16 |
| 40 - 80 | 4.12 | 97.52 |
| 80 - 160 | 8.10 | 93.40 |
| 160 - 320 | 16.00 | 85.30 |
| 320 - 640 | 23.00 | 69.30 |
| 640 - 1280 | 26.02 | 46.30 |
| 1280 - 2560 | 16.28 | 20.28 |
| 2560 - 5120 | 4.00 | 4.00 |
| >5120 | 0.00 | 0.00 |

**Example 4**

20 g of t-butylamine clavulanate is added to a mixture of isopropanol (50 ml), water (6 ml), methanol (12 ml), and acetic acid (0.1 ml), at about 20°C and stirred. The pH of the solution is adjusted to just below 7, with additional acetic acid as necessary. 1 g of charcoal is added to the solution, which is then stirred for 30 minutes, and then filtered over filter-aid, followed by washing with isopropanol (50 ml). The filtrate is added to a filtered mixture of 2N potassium ethyl hexanoate in isopropanol (47 ml), isopropanol (200 ml), and acetone (100 ml) maintained at 8-10°C over 15-20 minutes. The filter funnel is washed with isopropanol (30 ml) and the wash added to the suspension. The suspension is cooled to 0-5°C and stirred for 1 hour. The product is filtered, washed with two portions of acetone (50 ml each), sucked dry through a rubber sheet, and dried in a vacuum oven [120-125 Pa (27-28 inches Hg)] with a dry air bleed at 20°C for 18 hours.

The following data summarises results obtained by carrying out the above crystallisation procedure from a number of samples of t-butylamine clavulanate:

| | |
|---|---|
| Weight: | 14.7-15.0 g |
| Assay: | 82% (average; theoretical assay 83.9%) |
| Yield: | 90-94% |
| Crystal form: | Rosettes |

**Examples 5 - 7**

Three further samples of t-butylamine clavulanate were treated according to the procedure of Example 4 to give rosette-form potassium clavulanate.

Analysis results on the products are summarised in Table 5. The particle size of the products was determined by image analysis and the results are given in Table 6.

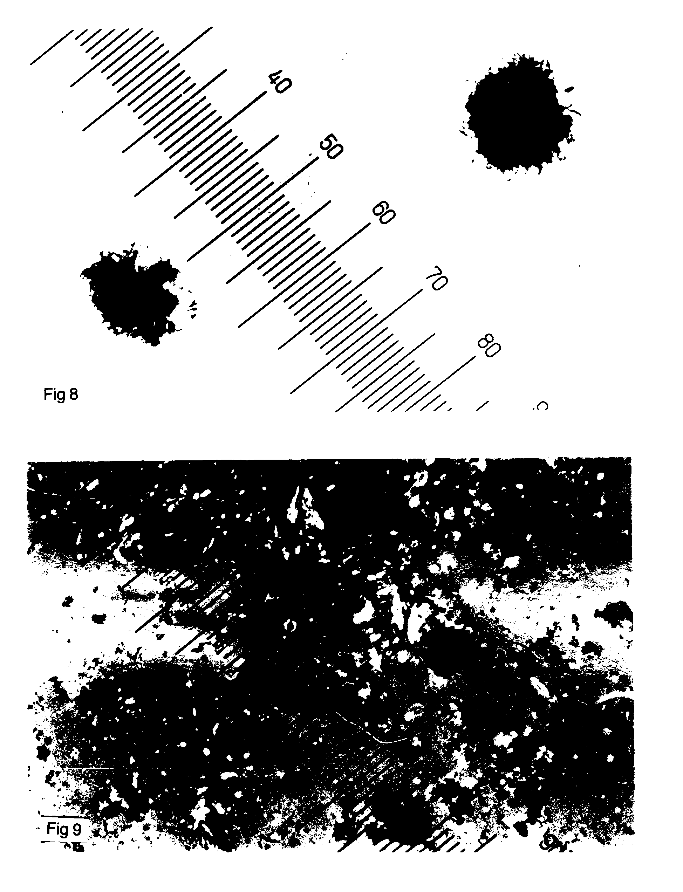

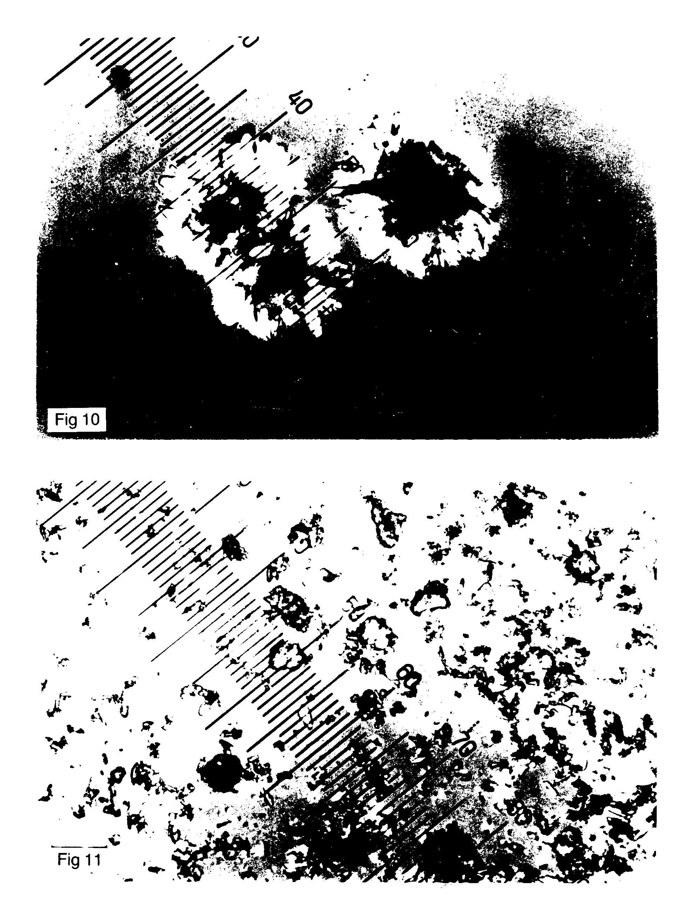

Fig. 8 shows a microphotograph of the product of Example 7 and Fig. 9 shows a microphotograph of a portion of the material of Example 7 which has been broken down by trituration with liquid paraffin in an agate pestle and mortar.

Table 5

| Analysis of rosette material (Examples 5 - 7) | | | |
|---|---|---|---|
| | Example 5 | Example 6 | Example 7 |
| Assay % | 81.84 | 81.61 | 81.75 |
| pH | 7.4 | 7.2 | 7.4 |
| Bulk density g/cm$^3$ | 0.75 | 0.77 | 0.53 |
| Acetone % | 0.24 | 0.32 | 0.24 |
| Methanol % | 0.11 | 0.12 | 0.10 |
| Isopropanol % | 0.25 | 0.32 | 0.25 |
| Water % | 0.30 | 0.28 | 0.25 |
| 2-ethyl hexanoic acid % | 0.58 | 0.59 | 0.43 |
| Clavam 2-carboxylate % | <0.01 | <0.01 | <0.01 |
| t-butylamine % | <0.05 | <0.05 | <0.05 |
| Physical form | Hard compact rosettes with slight surface crystal growth. Difficult to fracture. Flowed freely. | Very hard compact rosettes with no visible surface crystal growth. Very difficult to fracture. Flowed freely. | Soft rosettes with visible surface crystal growth. Easily fractured. Flowed freely |

Table 6: Particle size of rosette material (Example 5-7)

| Particle size range (μm²) | Volume % within range | | | Volume % within range and above | | |
|---|---|---|---|---|---|---|
| | Example 5 | Example 6 | Example 7 | Example 5 | Example 6 | Example 7 |
| 160 - 320 | 0.01 | 0.00 | 0.00 | 99.96 | 99.95 | 99.95 |
| 320 - 640 | 0.01 | 0.01 | 0.01 | 99.95 | 99.95 | 99.95 |
| 640 - 1280 | 0.03 | 0.04 | 0.01 | 99.94 | 99.94 | 99.94 |
| 1280 - 2560 | 0.11 | 0.13 | 0.01 | 99.91 | 99.90 | 99.93 |
| 2560 - 5120 | 1.35 | 0.84 | 0.01 | 99.80 | 99.77 | 99.92 |
| 5120 - 10240 | 6.56 | 5.51 | 0.09 | 98.45 | 98.93 | 99.91 |
| 10240 - 20480 | 25.27 | 22.41 | 1.69 | 91.89 | 93.42 | 99.82 |
| 20480 - 40960 | 37.98 | 43.91 | 17.40 | 66.62 | 71.01 | 98.13 |
| 40960 - 81920 | 28.64 | 24.53 | 54.54 | 28.64 | 27.10 | 80.73 |
| 81920 - 163840 | 0.00 | 2.57 | 26.19 | 0.00 | 2.57 | 26.19 |
| >163840 | 0.00 | 0.00 | 0.00 | 0.00 | 0.00 | 0.00 |

## Example 8

1.54 kg of t-butylamine clavulanate (equivalent to 1.00 kg of free acid at 65% purity) is dissolved in a mixture of 5 litres of isopropanol and 0.7 litre of water. After complete dissolution, the solution is filtered and then cooled to 8-10°C. 7.8 litres of isopropanol is added to 3.52 litres of 2N potassium ethyl hexanoate in isopropanol (about 1.4 mole equivalents), all of which is cooled to 8-10°C. The cooled tert-butylamine clavulanate solution is then added to the cooled potassium ethyl hexanoate solution, and the resulting slurry is stirred for 2 hours while being cooled to 0-5°C, and then filtered. The filtrate is washed with isopropanol and then with acetone, after which it is dried.

The product was in the form of soft rosettes, showing crystal growth radiating from a relatively hard core. Fig. 10 shows a microphotograph of the product.

The rosettes were easily fractured by trituration with liquid paraffin in an agate pestle and mortar, to give a mass of small crystals, as shown in Fig. 11.

The product was free flowing, and had a bulk density of 0.35 g/cm³.

The particle size of the product was determined by image analysis and the results are given in Table 7.

Table 7

| Particle size of rosette material (Example 8) | | |
|---|---|---|
| Particle size range ($\mu m^2$) | Volume % within range | Volume % within range and above |
| 160 - 320 | 0.00 | 99.95 |
| 320 - 640 | 0.00 | 99.95 |
| 640 - 1280 | 0.01 | 99.95 |
| 1280 - 2560 | 0.04 | 99.94 |
| 2560 - 5120 | 1.90 | 99.90 |
| 5120 - 10240 | 24.88 | 98.00 |
| 10240 - 20480 | 58.84 | 73.12 |
| 20480 - 40960 | 13.69 | 14.28 |
| 40960 - 81920 | 0.59 | 0.59 |
| >81920 | 0.00 | 0.00 |

**Comparison Examples A and B**

Two samples of the conventional crystalline form of potassium clavulanate in large individual needles (which may, in some cases, be randomly formed into loosely bound aggregates) were subjected to particle size determination by image analysis. The results are given in Table 8.

Fig. 7 shows a microphotograph of the sample of comparison Example A.

Table 8

| Particle size of needle-form material (Comparison) | | | | |
|---|---|---|---|---|
| Particle size range ($\mu m^2$) | Volume % within range | | Volume % within range and above | |
| | Example A | Example B | Example A | Example B |
| 10 - 20 | 0.83 | 1.94 | 99.97 | 99.93 |
| 20 - 40 | 2.64 | 3.41 | 99.14 | 97.99 |
| 40 - 80 | 5.73 | 4.91 | 96.50 | 94.58 |
| 80 - 160 | 9.65 | 8.02 | 90.77 | 89.67 |
| 160 - 320 | 14.51 | 13.40 | 81.12 | 81.65 |
| 320 - 640 | 15.98 | 16.01 | 66.61 | 68.25 |
| 640 - 1280 | 18.08 | 18.90 | 50.63 | 52.24 |
| 1280 - 2560 | 21.48 | 19.89 | 32.55 | 33.34 |
| 2560 - 5120 | 2.89 | 3.51 | 11.07 | 13.45 |
| 5120 - 10240 | 8.18 | 9.94 | 8.18 | 9.94 |
| >10240 | 0.00 | 0.00 | 0.00 | 0.00 |

**Claims**

1. A process for the preparation of rosette-form crystalline potassium clavulanate, characterised by adding a solution of clavulanate ions to a non-solvent for potassium clavulanate, in the presence of potassium ions.

2. A process according to claim 1, characterised in that precipitation of the crystals of potassium clavulanate is effected at a temperature not exceeding 15°C.

3. A process according to claim 1 or 2, characterised in that the clavulanate solution is of a pH within the range of from 6.5 to 7.0.

**4.** A process according to any one of claims 1, 2 or 3, characterised in that a solution of potassium clavulanate is added to a non-solvent for potassium clavulanate.

**5.** A process according to any one of claims 1, 2 or 3, characterised in that a solution of tert-butylamine clavulanate is added to a non-solvent for potassium clavulanate, in the presence of potassium ions.

**6.** A process according to claim 5, characterised in that the potassium ions are provided by potassium ethyl hexanoate.

**7.** A process according to any one of claims 1 to 6, characterised in that the solvent for the clavulanate ions is an aqueous alcohol.

**8.** A process according to claim 7, characterised in that the alcohol is methanol, ethanol or isopropanol, or a mixture of any two or more thereof.

**9.** A process according to any one of claims 1 to 8, characterised in that the non-solvent for potassium clavulanate is isopropanol or a mixture thereof with acetone.

**10.** A process according to any one of claims 5 or 6, characterised in that tert-butylamine clavulanate is dissolved in an aqueous alcohol selected from isopropanol, methanol or mixtures thereof, and the resulting solution is added to a solution of potassium ions in a solvent selected from isopropanol or a mixture thereof with acetone.

**11.** A process for the recrystallisation of potassium clavulanate, characterised by dissolving potassium clavulanate in aqueous methanol, aqueous ethanol or aqueous isopropanol, and admixing the resulting solution with a mixture of isopropanol and acetone, or in the case when the dissolution is carried out in aqueous iso-propanol alternatively with isopropanol, so as to cause precipitation of the potassium clavulanate.

**12.** A process according to claim 11, wherein the precipitation is carried out at a temperature within the range of from 10 to 15°C.

**13.** A process according to claim 11, wherein the precipitation is carried out at a temperature within the range of from 0 to 10°C.

**Patentansprüche**

**1.** Verfahren zur Herstellung rosettenförmigen, kristallinen Kaliumclavulanats, gekennzeichnet durch Zugeben einer Lösung von Clavulanationen zu einem Lösemittel, in dem sich Kaliumclavulanat nicht löst, in Gegenwart von Kaliumionen.

**2.** Verfahren nach Anspruch 1, dadurch gekennzeichnet, daß das Ausfällen der Kaliumclavulanatkristalle bei einer Temperatur bewirkt wird, die 15°C nicht übersteigt.

**3.** Verfahren nach Anspruch 1 oder 2, dadurch gekennzeichnet, daß die Clavulanatlösung einen pH-Wert innerhalb des Bereichs von 6,5 bis 7,0 aufweist.

**4.** Verfahren nach einem der Ansprüche 1, 2 oder 3, dadurch gekennzeichnet, daß eine Lösung des Kaliumclavulanats zu einem Lösemittel, in dem sich Kaliumclavulanat nicht löst, gegeben wird.

**5.** Verfahren nach einem der Ansprüche 1, 2 oder 3, dadurch gekennzeichnet, daß eine Lösung des tert.-Butylaminclavulanats zu einem Lösemittel, in dem sich Kaliumclavulanat nicht löst, in Gegenwart von Kaliumionen gegeben wird.

**6.** Verfahren nach Anspruch 5, dadurch gekennzeichnet, daß die Kaliumionen durch Kaliumethylhexanoat bereitgestellt werden.

12

**7.** Verfahren nach einem der Ansprüche 1 bis 6, dadurch gekennzeichnet, daß das Lösemittel für die Clavulanationen ein wäßriger Alkohol ist.

**8.** Verfahren nach Anspruch 7, dadurch gekennzeichnet, daß der Alkohol Methanol Ethanol oder Isopropanol oder ein Gemisch zweier oder mehrerer davon ist.

**9.** Verfahren nach einem der Ansprüche 1 bis 8, dadurch gekennzeichnet, daß das Lösemittel, in dem sich Kaliumclavulanat nicht löst, Isopropanol oder ein Gemisch davon mit Aceton ist.

**10.** Verfahren nach einem der Ansprüche 5 oder 6, dadurch gekennzeichnet, daß das tert-Butylaminclavulanat in einem wäßrigen Alkohol, gewählt aus Isopropanol, Methanol oder Gemischen davon, gelöst wird und die entstandene Lösung zu einer Lösung von Kaliumionen in einem Lösemittel, gewählt aus Isopropanol oder einem Gemisch davon mit Aceton, gegeben wird.

**11.** Verfahren zur Umkristallisation von Kaliumclavulanat, gekennzeichnet durch Lösen des Kaliumclavulanats in wäßrigem Methanol, wäßrigem Ethanol oder wäßrigem Isopropanol und Mischen der entstandenen Lösung mit einem Gemisch von Isopropanol und Aceton, oder in einer anderen Ausführungsform, falls die Auflösung in wäßrigem Isopropanol durchgeführt wird, in Isopropanol, um das Ausfallen des Kaliumclavulanats zu bewirken.

**12.** Verfahren nach Anspruch 11, wobei die Abscheidung bei einer Temperatur innerhalb eines Bereichs von 10 bis 15 °C durchgeführt wird.

**13.** Verfahren nach Anspruch 11, wobei die Abscheidung bei einer Temperatur innerhalb eines Bereichs von 0 bis 10 °C durchgeführt wird.

**Revendications**

**1.** Procédé pour la préparation de clavulanate de potassium cristallisé en forme de rosette, caractérisé par l'addition d'une solution d'ions clavulanate à un non solvant du clavulanate de potassium, en présence d'ions potassium.

**2.** Procédé selon la revendication 1, caractérisé en ce que la précipitation des cristaux de clavulanate de potassium est effectuée à une température n'excédant pas 15 °C.

**3.** Procédé selon la revendication 1, ou la revendication 2, caractérisé en ce que la solution de clavulanate est à un pH situé dans l'intervalle 6,5 à 7,0.

**4.** Procédé selon l'une quelconque des revendications 1, 2, ou 3, caractérisé en ce que la solution de clavulanate de potassium est additionnée à un non solvant du clavulanate de potassium.

**5.** Procédé selon l'une quelconque des revendications 1, 2, ou 3, caractérisé en ce qu'une solution de clavulanate de ter-butylamine est additionnée à un non solvant du clavulanate de potassium, en présence d'ions potassium.

**6.** Procédé selon la revendication 5, caractérisé en ce que les ions potassium sont fournis par l'éthylhexanoate de potassium.

**7.** Procédé selon l'une quelconque des revendications 1 à 6, caractérisé en ce que le solvant des ions clavulanate est un alcool aqueux.

**8.** Procédé selon la revendication 7, caractérisé en ce que l'alcool est le méthanol, l'éthanol, ou l'isopropanol, ou un mélange de deux quelconques d'entre eux, ou plus.

**9.** Procédé selon l'une quelconque des revendications 1 à 8, caractérisé en ce que le non solvant du clavulanate de potassium est l'isopropanol, ou un mélange de celui-ci avec l'acétone.

**10.** Procédé selon l'une quelconque des revendications 5 ou 6, caractérisé en ce que le clavulanate de ter-butylamine est dissous dans un alcool aqueux choisi parmi l'isopropanol, le méthanol, ou des mélanges de ceux-ci, et que la solution obtenue est additionnée à une solution d'ions potassium dans un solvant choisi parmi l'isopropanol ou un mélange de celui-ci avec l'acétone.

**11.** Procédé de recristallisation du clavulanate de potassium caractérisé par la dissolution du clavulanate de potassium dans le méthanol aqueux, l'éthanol aqueux, ou l'isopropanol aqueux, et le mélange de la solution obtenue, avec un mélange d'isopropanol et d'acétone, ou, au cas où la dissolution est effectuée dans l'isopropanol aqueux, en alternative, avec l'isopropanol, de façon à provoquer la précipitation du clavulanate de potassium.

**12.** Procédé selon la revendication 11, dans lequel la précipitation est effectuée à une température située dans l'intervalle compris entre 10 et 50 °C.

**13.** Procédé selon la revendication 11, dans lequel la précipitation est effectuée à une température située dans l'intervalle compris entre 0 et 10 °C.

FIG. 2

FIG. 4

FIG. 1

FIG. 3

FIG. 5

FIG. 6

FIG. 7

Fig 8

Fig 9

Fig 10

Fig 11